# EUROPÄISCHE PATENTSCHRIFT

(11) **EP 1 237 834 B1**
(45) Veröffentlichungstag und Bekanntmachung des Hinweises auf die Patenterteilung: **14.01.2004**
(21) Anmeldenummer: 00974481.4
(22) Anmeldetag: 30.10.2000
(51) Int. Cl.: C07C 39/16

(54) **BISPHENOL-HERSTELLUNG**
BISPHENOL PRODUCTION
PRODUCTION DE BISPHENOL

(30) Priorität: 11.11.1999 DE 19954311
(43) Veröffentlichungstag der Anmeldung: 11.09.2002
(73) Patentinhaber: Bayer Aktiengesellschaft, 51368 Leverkusen (DE)
(72) Erfinder: HEYDENREICH, Frieder, 40593 Düsseldorf (DE); PREIN, Michael, 47802 Krefeld (DE); BÖDIGER, Michael, 41539 Dormagen (DE); NEUMANN, Rainer, 47803 Krefeld (DE); FENNHOFF, Gerhard, 2940 Stabroek (BE); VAES, Johan, 2920 Kalmthout (BE)
(86) Internationale Anmeldenummer: PCT/EP2000/010655
(87) Internationale Veröffentlichungsnummer: WO 2001/034544

(56) Entgegenhaltungen:
- EP-A- 0 758 637

## Beschreibung

Die Erfindung betrifft ein Verfahren zur effizienten Herstellung von hochreinen Bisphenolen.

Bisphenole sind wichtige Rohstoffe für die Herstellung von Polymeren wie Epoxy-Harze oder insbesondere Polycarbonate. Für diesen Einsatz werden große Anforderungen an die Reinheit der Bisphenole gestellt, so dass für ökonomische, großtechnische Herstellungsverfahren neben der Erzielung hoher Umsätze und Selektivitäten in der Reaktion insbesondere den Aufarbeitungsschritten Bedeutung zukommt.

Bisphenole werden nach prinzipiell bekannten Verfahren durch die Kondensation von Carbonylverbindungen mit aromatischen Alkoholen in Gegenwart von sauren Katalysatoren hergestellt. Ein Verfahren für die technisch bedeutende Herstellung von 2,2-Bis(4-hydroxyphenyl)propan (BPA) ist die Umsetzung von Aceton und Phenol in Gegenwart von vernetzten sulfonierten Polystyrolharzen (Ionentauscherharzen). Hierbei wird ein Phenol-/Acetonverhältnis von mindestens 5 : 1 eingestellt. Zur Erzielung hoher Selektivitäten kommen Cokatalysatoren zum Einsatz, die entweder homogen in den Reaktanden gelöst sind oder durch kovalente oder ionische Bindungen am Ionentauscherharz fixiert sind.

Aufgabe ist, die in dem obigen Verfahren zur Herstellung von BPA entstehenden Nebenprodukte durch geeignete Maßnahmen von BPA abzutrennen und überschüssiges Phenol restlos aus dem Produkt zu entfernen. Außerdem sollten die bei diesen Maßnahmen entstehenden Seitenströme auf ökonomischen Wege im Gesamtprozess wiederverwertet werden.

Als Lösung dieser Aufgaben wird in der Literatur die Isolierung von BPA-Phenol-Adduktkristallen aus der Reaktionslösung durch Suspensionskristallisation mit oder ohne vorherige Destillation beschrieben (EP-A-829464), wobei die bei der Filtration der Adduktkristalle entstehende stark phenolhaltige Mutterlauge, gegebenenfalls nach Einschaltung einer Umlagerungsreaktion, vor die Reaktionseinheit zurückgeführt und mit frischem Phenol und Aceton ergänzt wird. Nachteil dieses Vorgehens ist die Generierung großer Kreislaufströme im Verfahren und die Kontamination des Katalysatorbettes durch die Rückführung des stark verunreinigten Mutterlaugenstroms.

Zur Umgehung dieser Problematik wurden Verfahren vorgeschlagen, die auf eine Adduktkristallisation und somit die Erzeugung eines Mutterlaugenkreislaufs verzichten. In EP-A-758637 ist die Herstellung von BPA durch Aufarbeitung des Reaktionsstroms in einer Kaskade destillativer Reinigungsschritte beschrieben, wobei der Reaktionsstrom ohne Generierung von Rückströmen von Aceton, Wasser, Phenol und Nebenprodukten gereinigt wird. Nachteile dieses Verfahrens sind die thermische Belastung des Produkts durch die hohen Temperaturen bei der Destillation, die hohen Energiekosten der Destillationskaskade und der hohe Verlust an Rohstoffen durch den Verzicht auf Umlagerung und Rückführung von Nebenkomponenten.

In EP-A-785181 erfolgt die Aufarbeitung der Reaktionslösung ebenfalls unter Verzicht auf eine Adduktkristallisation durch eine Kombination von Vakuumdestillation zur Entfernung von Aceton, Wasser, Phenol und gegebenenfalls Nebenkomponenten und anschließende Schmelzekristallisation. Auch bei diesem Vorgehen sind durch den Verzicht auf eine Rückführung von BPA-haltigen Nebenproduktströmen, die bei der Reinigung entstehen, Einbußen an Rohstoffen hinzunehmen.

Das erfindungsgemäße Verfahren zur Herstellung und Aufarbeitung von BPA umgeht die oben beschriebenen Nachteile, indem die Reaktionsmischung mittels Vakuumdestillation gereinigt, anschließend eine Schichtkristallisation durchgeführt wird und die Nebenproduktströme zurückgeführt werden. Mit dem erfindungsgemäßen Verfahren kann hochreines BPA hergestellt werden. Durch die Rückführung der Nebenproduktströme hinter die Produktionseinheit wird der Katalysator in der Reaktionseinheit nicht durch rückgeführte Nebenprodukte belastet.

Gegenstand der Erfindung ist somit ein Verfahren zur Herstellung von 2,2-Bis(4-hydroxyphenyl)propan (BPA) durch Umsetzung von Aceton und Phenol in Gegenwart von vemetzten sulfonierten Polystyrolharzen (Ionentauscherharzen), dadurch gekennzeichnet, dass im Anschluss an die Reaktionseinheit, BPA aus der Reaktionslösung
a) durch eine mehrstufige Vakuumdestillation,
b) eine anschließende ein- oder mehrstufige Schichtkristallisation, abgetrennt,
c) und der an Nebenprodukten angereicherte Nebenproduktstrom, hinter die Reaktionseinheit zurückgeführt wird.

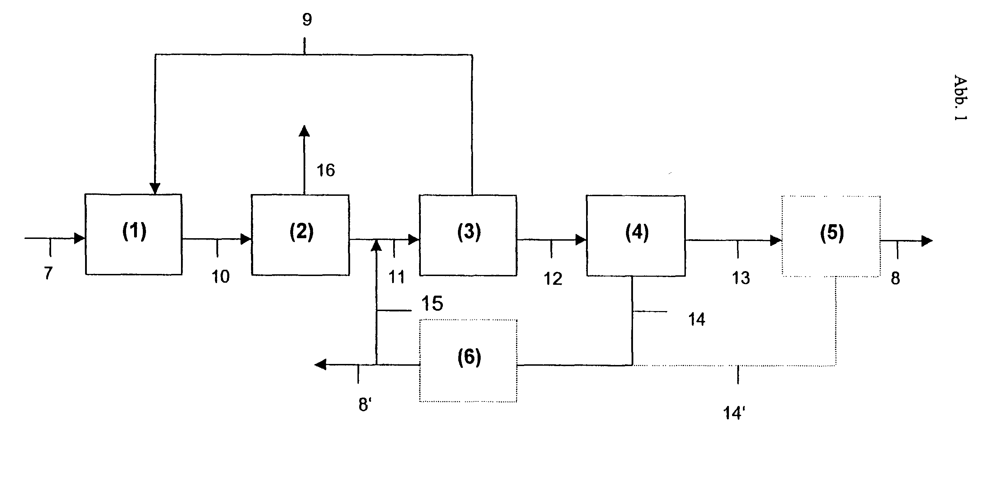

Das erfindungsgemäße Verfahren wird in dem Verfahrensschema Abb. 1 näher erläutert.

(1) stellt eine Reaktionseinheit dar, in der Phenol, Aceton mit einem Phenol/Acetonverhältnis von mindestens 5 : 1, bevorzugt mindestens 10 : 1 bei Temperaturen von 40 bis 110°C, bevorzugt 45 bis 70°C, einem Ionenaustauscher-Katalysatorsystem, bestehend aus einem sulfonierten vernetzten Polystyrol, zugeführt werden.

Das Ionentauscher-Katalysatorsystem ist bevorzugt entweder mit einer kovalent oder ionisch gebundenen Mercaptoverbindung modifiziert.

Die Reaktionseinheit ist beispielhaft und vorzugsweise ein Schichtbett oder Wirbelbett, die auf- oder abwärts durchflossen werden, oder eine Kolonne nach Art einer Reaktivdestillationskolonne.

Der aus der Reaktionseinheit (1) austretende Reaktionsstrom (Reaktionslösung) enthält nicht umgesetztes Phenol und Aceton, daneben Wasser, BPA und die bei der Reaktion typischerweise entstehenden Nebenkomponenten wie o,p-BPA, Indane, Chromane, und höher kondensierte Reaktionsprodukte mit drei oder mehr aromatischen Kernen.

Die Reaktion wird so geführt, dass die die Reaktionseinheit (1) verlassende Reaktionsmischung 70 bis 95 Gew.%, bevorzugt 75 bis 90 Gew.% Phenol; 0 bis 4 Gew.%, bevorzugt 0,1 bis 1,0 Gew.% Aceton; 0,1 bis 4 Gew.% bevorzugt 0,5 bis 2,0 Gew.% Wasser; 5 bis 30 Gew.%, bevorzugt 10 bis 25 Gew.% p,p-BPA und 0 bis 3 Gew.%, bevorzugt 0,1 bis 1,5 Gew.% Nebenprodukte aufweist.

Dieser Reaktionsstrom wird einer Kaskade von zwei Destillationskolonnen (2) und (3) zugeführt. In der Destillationskolonne (2) wird aus dem Reaktionsstrom Wasser und Aceton bis zu einem Restgehalt von jeweils nicht mehr als 0,1 Gew.% entfernt.

In der zweiten Destillationskolonne (3) wird dann Phenol bis zu einem Restgehalt von nicht mehr als 25 Gew.%, bevorzugt nicht mehr als 10 Gew.%, besonders bevorzugt nicht mehr als 5 % abgetrennt. Das abgetrennte Phenol wird vorzugsweise in die Reaktionseinheit (1) zurückgeführt.

Der verbleibende, neben BPA noch Nebenkomponenten und Rest-Phenol enthaltende Reaktionsstrom wird einer Kristallisationseinheit (4) zugeführt. In der Kristallisationseinheit (4) wird die Kristallisation als Schichtkristallisation an gekühlten Oberflächen in einer kontinuierlich oder diskontinuierlich betriebenen Schmelzekristallisationapparatur bei Temperaturen von 125 bis 160°C, bevorzugt 140 bis 160°C durchgeführt. Die Kristallisation kann statisch oder in Form einer Fallfilmkristallisation durchgeführt werden.

Durch diese Verfahrensführung wird eine Anreicherung von BPA und eine Abreicherung der Nebenkomponenten und von Phenol erreicht. Der Phenolgehalt und der Nebenkomponentengehalt wurde in der Kristallisationseinheit (4), gegenüber der vor Eintritt in die Kristallisationseinheit (4) vorhandenen Mengen, jeweils um ca. 80% verringert.

Nach Beendigung des Kristallwachstums werden die gebildeten BPA-Kristalle durch Ablassen der flüssigen Mutterlauge isoliert. Gegebenenfalls wird anschließend eine weitere Reinigung der Kristalle, beispielhaft und vorzugsweise mittels Durchlaufen eines Temperaturgradienten durch Schwitzen, durchgeführt. Abschließend werden die Kristalle durch Erhöhung der Temperatur über den Schmelzpunkt verflüssigt und zur weiteren Verarbeitung in einen Sammelbehälter abgelassen.

Das nach der Kristallisationseinheit (4) erhaltene BPA kann gegebenenfalls in einem zweiten Einheit (5) von noch vorhandenem Phenol und/oder Nebenkomponenten befreit werden. Die Einheit (5) wird dabei vorzugsweise wie die Kristallisationseinheit (4) betrieben. Die Reinigung kann aber auch durch Destillation und/oder Desorption erfolgen.

Der in der Kristallisationseinheit (4) bzw. gegebenenfalls (5) erhaltene Nebenproduktstrom, der BPA, Phenol und angereicherte Nebenkomponenten enthält, wird vor die zweite Destillationskolonne (3) zurückgeführt. Dabei kann ein Teil des (Neben)Produktes (BPA-Harz) aus dem System entfernt werden, um eine Akkumutation von Nebenkomponenten im Kreislauf zu verhindern.

Vorzugsweise wird der Nebenproduktstrom bevor er in die Destillationskolonne (3) eingespeist wird, über einen Umlagerungsreaktor (6) geführt, der mit saurem Ionentauscher, vorzugsweise mit vernetzten sulfonierten Polystyrolharzen, insbesondere mit kovalent oder ionisch gebundenen Mercaptoverbindungen, insbesondere mit 2-Mercaptoethylamin (Cysteamin) modifizierten und vernetzten sulfonierten Polyesterharzen befüllt ist und bei Temperaturen von 50 bis 110°C, bevorzugt 60 bis 80°C betrieben wird. Im Umlagerungsreaktor (6) werden einige der im Nebenproduktstrom enthaltenen Nebenprodukte (o,p-BPA, höhere Kondensate) in p,p-BPA umgelagert. So kann die Gesamtausbeute an BPA erhöht und der BPA-Harz-Anteil verringert werden.

Im Verfahrensschema Abb. 1 steht desweiteren
- (7): Zuführungseinheit für Phenol und Aceton
- (8) und (8'): für eine Produktentnahmeeinheit
- (9): für eine Phenol-Rückführleitung
- (10) bis (13): für Reaktionsstrom-Leitungen
- (14) und (14'): für Nebenproduktstrom-Leitungen
- (15): für eine Nebenproduktstrom- bzw. Umlagerungsproduktstrom-Leitung
- (16): für eine Wasser und Aceton-Abführleitung

Bei dem erfindungsgemäßem Verfahren wird qualitativ hochwertiges BPA in ökonomischer Weise unter Minimierung von Rückströmen, Energieeintrag und Stoffverlusten erhalten ohne dass der Katalysator in der Reaktionseinheit (1) mit zurückgeführten Nebenprodukten belastet und deaktiviert wird. Durch Einsatz der Schichtkristallisation kann außerdem auf den Einsatz von Drehfiltem und Zentrifugen zur Abtrennung von Adduktkristallen verzichtet werden die ansonsten bei einer Suspensionskristallisation von BPA anfallen würden. Somit wird der technisch aufwendige, wartungsintensive Betrieb von Prozessapparaturen vermieden und die Verfügbarkeit der Anlage erhöht. Das auf diesem Wege hergestellte BPA ist zur Nutzung als Rohstoff für Polymere wie insbesondere Polycarbonate oder Epoxy-Harze aufgrund seiner hohen Reinheit und guten Farbzahl besonders geeignet.

Die nachfolgenden Beispiele dienen zur Erläuterung der Erfindung. Die Erfindung ist nicht auf die Beispiele limitiert. Prozentangaben bedeuten nachfolgend Gewichtsprozente.

### Beispiele

### Beispiel 1

### Herstellung Reaktionslösung

In einem doppelwandigem Glasreaktor (5 1), werden über einer Glasfritte 4 1 phenolfeuchtes sulfoniertes Polystyrolharz (Lewatit SC 104, Bayer AG, belegt mit 5 % Cysteamin) vorgelegt. Die Apparatur wird auf 70°C thermostatisiert und durch 5-maliges Evakuieren und Belüften mit Stickstoff inertisiert. Unter Stickstoffbeschleierung wird kontinuierlich eine Lösung von 96 % Phenol und 4.0 % Aceton bei 70°C und einer Durchflussrate von 1.5 1/h von oben in den Reaktor eingespeist. Am Ablauf des Reaktors erhält man im kontinuierlichen Betrieb eine Lösung der folgenden Zusammensetzung: Phenol 84.5 %, Aceton 0.3 %, Wasser 1.4 %, p,p-BPA 12.7 %, Nebenprodukte 1.1 %

### Beispiel 2

### Entfernung Aceton/Wasser aus Reaktionslösung

Die in Beispiel 1 erhaltene Reaktionslösung (10 l) wird über zwei Plattenkolonnen mit 10 Trennstufen unter Stickstoffbeschleierung destilliert. Die Destillation erfolgt bei 150 mbar und 130°C Bodentemperatur. Man erhält ein Destillat folgender Zusammensetzung:

Aceton 17.0 %, Wasser 79.3 %, Phenol 3.6 %, Sonstige < 0.1 %

Das Bodenprodukt zeigt folgende Zusammensetzung: Phenol 85.9 %, p,p-BPA 12.9 %, Nebenprodukte 1.2 %

### Beispiel 3

### Phenolentfernung aus der entwässerten Lösung

Die Phenoldestillation der in Beispiel 2 erhaltenen Lösung erfolgte in einer Füllkörperkolonne bei 100 mbar, die Kopftemperatur betrug 120°C. Das Destillat enthält >99.9 % Phenol, das Bodenprodukt zeigt folgende Zusammensetzung: Phenol 4.0 %, p,p-BPA 88.3 %, Nebenkomponenten 7.7 %

### Beispiel 4

### Schichtkristallisation

Die in Beispiel 3 erhaltene Lösung wird einer statischen Schichtkristallisation in einer Schmelzekristallisationsanlage bei 148 bis 153°C zugeführt. Nach Kristallwachstum, Ablassen der Mutterlauge, Waschen der Kristalle mit Reaktionslösung und Abschwitzen der Kristalle werden die Produktkristalle bei 165°C aufgeschmolzen und abgelassen. Es wird ein Produkt der folgenden Zusammensetzung erhalten: Phenol: 0.7 %, p,p-BPA 98.2 %, Nebenprodukte 1.1 %.

Der so erhaltene Produkstrom wird einer zweiten Schichtkristallisation zugeführt, die bei 153-156°C betrieben wird. Nach Kristallwachstum, Ablassen der Mutterlauge, Waschen der Kristalle mit Reaktionslösung und Abschwitzen der Kristalle werden die Produktkristalle bei 165°C aufgeschmolzen und abgelassen. Es wird ein Produkt der folgenden Zusammensetzung erhalten: Phenol: 0.1 %, p,p-BPA 99.7 %, Nebenprodukte 0.2 %.

Die Abreicherung an Phenol und Nebenprodukten in den beiden Stufen errechnet sich wie folgt:
Schichtkristallisation 1:
   Phenol Reduktion um 83 %, Nebenprodukte Reduktion um 86 %
Schichtkristallisation 2:
   Phenol Reduktion um 86 %, Nebenprodukte Reduktion um 82 %

Die Gesamtmenge an rückgeführter Mutterlauge über beide Kristallisationsstufen betrug 18 % der eingesetzten Reaktionslösung.

## Patentansprüche

1. Verfahren zur Herstellung von 2,2-Bis(4-hydroxyphenyl)propan (BPA) durch Umsetzung von Aceton und Phenol in Gegenwart von vernetzten sulfonierten Polystyrolharzen (Ionentauscherharzen), **dadurch gekennzeichnet, dass** im Anschluss an die Reaktionseinheit, BPA aus der Reaktionslösung
a) durch eine mehrstufige Vakuumdestillation,
b) eine anschließende ein- oder mehrstufige Schichtkristallisation, abgetrennt,
c) und der an Nebenprodukten angereicherte Nebenproduktstrom hinter die Reaktionseinheit zurückgeführt wird.

2. Verfahren nach Anspruch 1, **dadurch gekennzeichnet, dass** der rückgeführte Nebenproduktstrom über einen mit vernetzten sulfonierten Polysystrolharz (sauren Ionentauscherharze) befüllten Umlagerungsreaktor geführt wird.

3. Verfahren nach Anspruch 1 oder 2, dadurch gekenzeichnet, dass das zur Herstellung verwendete vernetzte sulfonierte Polystyrolharz mit einer kovalent oder ionisch gebundenen Mercaptoverbindung modifiziert ist.

4. Verfahren nach Anspruch 3, **dadurch gekennzeichnet, dass** die ionisch gebundene Mercaptoverbindung 2-Mercaptoethylamin (Cysteamin) ist.

5. Vorrichtung **gekennzeichnet durch** die Kombination einer Reaktionseinheit (1) mit einer Destillationseinheit (2), einer Destillationseinheit (3) und mindestens einer Schichtkristallisationseinheit (4) bzw. (5) und gegebenenfalls einem Umlagerungsraktor (6).

6. Verwendung der Vorrichtung wie in Anspruch 5 definiert zur Herstellung von BPA.

## Claims

1. Process for the production of 2,2-bis(4-hydroxyphenyl)propane (BPA) by reaction of acetone and phenol in the presence of cross-linked sulfonated polystyrene resins (ion exchanger resins), **characterised in that** BPA is separated from the reaction solution downstream of the reaction unit
a) by means of a multi-stage vacuum distillation and
b) a subsequent single- or multi-stage layer crystallisation,
c) and the by-product stream with accumulated by-products is returned downstream of the reaction unit.

2. Process according to claim 1, **characterised in that** the recycled by-product stream is passed through a rearrangement reactor filled with cross-linked sulfonated polystyrene resin (acid ion exchanger resins).

3. Process according to claim 1 or 2, **characterised in that** the cross-linked sulfonated polystyrene resin used for the production is modified with a covalently or ionically bonded mercapto compound.

4. Process according to claim 3, **characterised in that** the ionically bonded mercapto compound is 2-mercaptoethylamine (cysteamine).

5. Arrangement **characterised by** the combination of a reaction unit (1) with a distillation unit (2), a distillation unit (3) and at least one layer crystallisation unit (4) and/or (5) and optionally a rearrangement reactor (6).

6. Use of the arrangement as defined in claim 5 for the production of BPA.

## Revendications

1. Procédé pour la préparation du 2,2-bis-(4-hydroxyphényl)-propane (BPA) par réaction de l'acétone et du phénol en présence de résines de polystyrène sulfonées et réticulées (résines échangeuses d'ions), **caractérisé en ce que**, après l'unité de réaction, on sépare le BPA de la solution de réaction
a) par une distillation sous vide à plusieurs étages,
b) une cristallisation subséquente en couche à un ou plusieurs étages, et
c) on recycle le courant de produits d'accompagnement appauvri en ces derniers en aval de l'unité de réaction.

2. Procédé selon la revendication 1, **caractérisé en ce que** le courant de produits d'accompagnement recyclé est envoyé dans un réacteur de transposition garni d'une résine de polystyrène sulfonée et réticulée (résine échangeuse d'ions acides).

3. Procédé selon la revendication 1 ou 2, **caractérisé en ce que** la résine de polystyrène sulfonée et réticulée utilisée pour la préparation est modifiée par un mercaptan fixé par des liaisons covalentes ou ioniques.

4. Procédé selon la revendication 3, **caractérisé en ce que** le mercaptan fixé par des liaisons ioniques est la 2-mercaptoéthylamine (cystéamine).

5. Appareillage **caractérisé par** la combinaison d'une unité de réaction (1) avec une unité de distillation (2), une unité de distillation (3) et au moins une unité de cristallisation en couche (4) ou (5) et le cas échéant un réacteur de transposition (6).

6. Utilisation de l'appareil défini dans la revendication 5 pour la préparation du BPA.
